(19) Europäisches Patentamt
European Patent Office
Office européen des brevets

(11) Veröffentlichungsnummer: **0 020 950**
**B1**

(12) # EUROPÄISCHE PATENTSCHRIFT

(45) Veröffentlichungstag der Patentschrift: **12.05.82**

(51) Int. Cl.³: **C 07 C 121/30,** C 07 C 121/70

(21) Anmeldenummer: **80102411.8**

(22) Anmeldetag: **05.05.80**

(54) **Verfahren zur Herstellung von Alpha, Beta-ungesättigten Nitrilen aus Aldehyden und Formamid.**

(30) Priorität: **16.05.79 DE 2919630**

(43) Veröffentlichungstag der Anmeldung: **07.01.81 Patentblatt 81/1**

(45) Bekanntmachung des Hinweises auf die Patenterteilung: **12.05.82 Patentblatt 82/19**

(84) Benannte Vertragsstaaten: **BE CH DE FR GB LI NL**

(56) Entgegenhaltungen: **CH-A-391 681**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Hutmacher, Hans-Martin, Dr., Neckarpromenade 16, D-6800 Mannheim 1 (DE)**
Erfinder: **Hagen, Helmut, Dr., Max-Slevogt-Strasse 17E, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 020 950 B1

Verfahren zur Herstellung von $\alpha,\beta$-ungesättigten Nitrilen aus Aldehyden und Formamid

Die Erfindung betrifft ein Verfahren zur Herstellung von $\alpha,\beta$-ungesättigten Nitrilen durch Umsetzung von Aldehyden mit Formamid in Gegenwart von Säure und gegebenenfalls nichtaromatischer Lösungsmittel zu N-Alkenylformamiden und deren Umsetzung in der Gasphase an Dehydratationskatalysatoren bei erhöhter Temperatur.

Es ist bekannt, $\alpha,\beta$-ungesättigte Nitrile durch katalytische Dehydrierung gesättigter Nitrile bei 500 bis 580° C durchzuführen (DE-PS 1 127 890) herzustellen. Weiterhin ist bekannt, gesättigte Nitrile durch Umsetzung und Umlagerung von N-formylierten, primären Monoaminoverbindungen an Kieselsäurekatalysatoren spezieller Struktur herzustellen (DE-AS 1 117 121). In den Beispielen werden Reaktionstemperaturen von 400 bis 540°C aufgeführt. In einer Verfahrensvariante (DE-OS 2 036 503) werden $\alpha,\beta$-ungesättigte Nitrile über die schwierig und nur in begrenztem Rahmen herstellbaren N-formylierten Allylamine durch Wasserabspaltung und Isomerisierung bei Temperaturen zwischen 500 und 800° C erhalten.

Es ist aus dem Archiv der Pharmazie, Band 299 (1966), Seiten 493 bis 498, bekannt, daß man Formamid mit Diphenylacetaldehyd in Gegenwart eines sauren Katalysators zu N-($\beta,\beta$-Diphenylvinyl)-formamid umsetzt, wenn man das Gemisch am Wasserabscheider während 50 Stunden mit Benzol als Schleppmittel erhitzt. Führt man die Umsetzung mit Amylalkohol als Schleppmittel durch, so erhält man 1,1,5,5-Tetraphenyl-3-azapentadien-(1,4).

CH-PS 391 681 lehrt die Herstellung von Nitrilen aus N-formylierten primären Aminen bei erhöhter Temperatur in der Gasphase in Gegenwart von Kieselsäure oder Sulfaten besonderer Struktur. Das Verfahren ist ausdrücklich auf N-formylierte primäre Amine beschränkt, was auch aus den verwendeten Ausgangsstoffen (Aminen) klar hervorgeht (Seite 1, Zeilen 45–57). Kein einziges Beispiel eines $\alpha,\beta$-ungesättigten Formamids ist beschrieben. Diese Feststellung ist besonders angesichts des Alters der Reaktion ein Vorurteil gegen den Einsatz sonstiger Formamide, d. h. der N-Alkenylformamide.

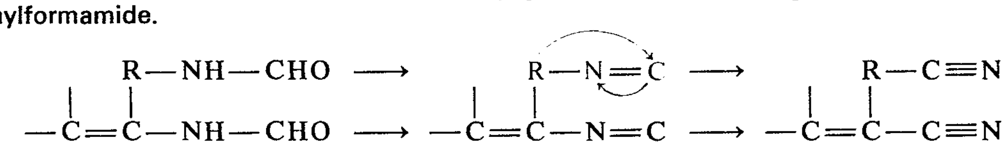
(1)
(2)

Es wurde nun gefunden, daß man $\alpha,\beta$-ungesättigte Nitrile der Formel

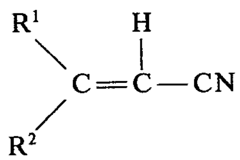
(I)

worin $R^1$ und $R^2$ gleich oder verschieden sein können und jeweils einen aliphatischen, cycloaliphatischen, araliphatischen, aromatischen oder heterocyclischen Rest bedeuten, $R^1$ und $R^2$ zusammen mit dem benachbarten Kohlenstoffatom auch Glieder eines Ringes bezeichnen, durch Umsetzung von Aldehyden mit Säureamiden in Gegenwart von sauren Katalysatoren und Lösungsmitteln vorteilhaft erhält, wenn man in einem ersten Schritt einen Aldehyd der Formel

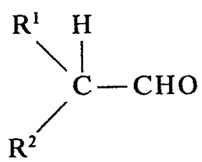
(II)

worin $R^1$ und $R^2$ die vorgenannten Bedeutungen besitzen, mit Formamid in Gegenwart von katalytischen Mengen einer anorganischen Säure, Sulfonsäure und/oder halogensubstituierten, aliphatischen Carbonsäure in Abwesenheit von zusätzlichen Lösungsmitteln oder in Gegenwart unter den Reaktionsbedingungen inerter, organischer, nichtaromatischer Lösungsmittel umsetzt und dann in einem zweiten Schritt das so gebildete N-Alkenylformamid der Formel

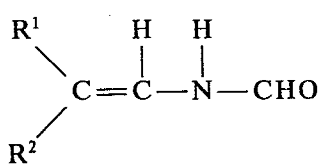
(III)

worin $R^1$ und $R^2$ die vorgenannten Bedeutungen besitzen, in der Gasphase bei einer Temperatur von

250 bis 700° C in Gegenwart eines Dehydratationskatalysators umsetzt.

Die Umsetzung kann im Falle der Verwendung von Isobutyraldehyd durch die folgenden Formeln wiedergegeben werden:

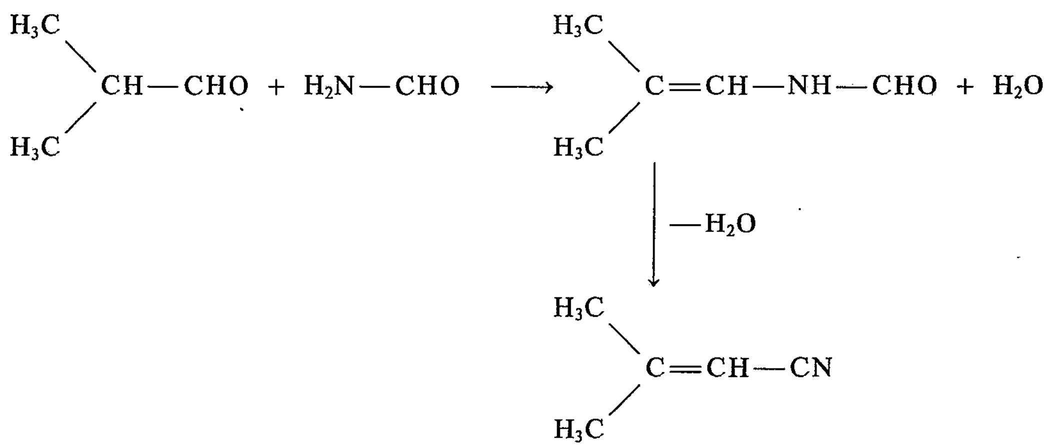

Im Vergleich zu den bekannten Verfahren liefert das Verfahren nach der Erfindung, ausgehend von leicht zugänglichen Ausgangsstoffen, auf einfacherem und wirtschaftlicherem Weg $\alpha,\beta$-ungesättigte Nitrile in besserer Ausbeute und Reinheit. Im Hinblick auf die Arbeit im Archiv der Pharmazie wird überraschend im allgemeinen bei wesentlich geringeren Reaktionszeiten umgesetzt. Alle diese vorteilhaften Eigenschaften sind im Hinblick auf den Stand der Technik überraschend. So konnte nicht erwartet werden, daß eine Umlagerung der intermediären Isonitrilgruppe zur Nitrilgruppe eintritt, da ja die Doppelbindung gerade in $\alpha,\beta$-Stellung und nicht in $\beta,\gamma$-Stellung im Molekül steht. Im Hinblick auf die genannte Arbeit im Archiv der Pharmazie (loc. cit.) mußte vermutet werden, daß die erfindungsgemäßen Reaktionsbedingungen unter Verwendung nichtaromatischer Lösungsmittel zur Bildung von Azapentadienderivaten oder zumindest zur Bildung von heterogenen Gemischen unterschiedlicher Kondensationsprodukte der Ausgangsstoffe führen würde.

Betrachtet man im Hinblick auf die Lehre der CH-PS 391 681 den allgemein anerkannten Mechanismus der Umlagerung (Gleichung 1), wonach Isonitrile Zwischenprodukte sind, so ist dieses Vorurteil dem Fachmann auch plausibel gewesen. Im Fall der N-Alkenylformamide (Gleichung 2) ist das Zwischenprodukt ein $\alpha,\beta$-ungesättigtes Isonitril, also theoretisch ein Dien, das sich thermodynamisch von einem z. B. Alkylisonitril wesentlich unterscheidet. Keineswegs ist hier die Wanderung einer Vinylgruppe, d. h. eine Umlagerung, bekannt noch zu erwarten gewesen.

Wie aus Synthesis 1978, 489, ersichtlich, haben Enamide bekanntlich eigenen Charakter. Über Umlagerungen ist nichts bekannt. Seite 494 zeigt, »The enamides are also stable thermally and there are no known rearrangements of simple enamides«.

Die Ausgangsstoffe II können mit Formamid in stöchiometrischer Menge oder im Überschuß jeder Komponente, bezogen auf die andere, vorzugsweise in einem Verhältnis zwischen 0,75 und 4, vorteilhaft von 0,76 bis 2, insbesondere 0,9 bis 1,5 Mol Ausgangsstoff II je Mol Formamid umgesetzt werden. Bevorzugte Ausgangsstoffe II und demzufolge bevorzugte Endstoffe I sind solche, in deren Formeln $R^1$ und $R^2$ gleich oder verschieden sein können und jeweils einen Alkylrest mit 1 bis 7 Kohlenstoffatomen, einen Cycloalkylrest mit 5 bis 7 Kohlenstoffatomen, einen Arylalkylrest oder Alkylarylrest mit 7 bis 12 Kohlenstoffatomen, einen Phenylrest oder einen 5- oder 6gliedrigen, heterocyclischen Rest, der ein oder 2 Stickstoffatome und/oder ein Sauerstoffatom enthalten kann, bedeuten, $R^1$ und $R^2$ zusammen mit dem benachbarten Kohlenstoffatom auch Glieder eines 5- oder 6gliedrigen, alicyclischen Ringes bezeichnen. Die vorgenannten Reste und Ringe können noch durch unter den Reaktionsbedingungen inerte Gruppen, z. B. Alkylgruppen oder Alkoxygruppen mit jeweils 1 bis 4 Kohlenstoffatomen, substituiert sein.

Beispielsweise sind folgende Aldehyde als Ausgangsstoffe II geeignet: In $\alpha$-Stellung zweifach durch die Methyl-, Äthyl-, Propyl-, Isopropyl-, Butyl-, Isobutyl-, sek.-Butyl-, tert.-Butyl, Cyclopentyl-, Cyclohexyl-, Benzyl-, Phenyl-, o-Tolyl-, m-Tolyl-, p-Tolyl-, o-Methoxyphenyl-, m-Methoxyphenyl-, p-Methoxyphenyl-, Pentyl-, Hexyl-, Heptyl-, Piperidinyl-(2)-gruppe gleich oder unterschiedliche substituierte Acetaldehyde; Cyclohexyl-, Cyclopentylaldehyd; bevorzugt sind Isobutyraldehyd, 2-Methylbutanal, 2-Methylpentanal, 2-Äthylhexanal, 2-Phenylpropanal.

Die Umsetzung des ersten Schrittes wird zweckmäßig bei einer Temperatur von 40 bis 150° C, vorteilhaft von 60 bis 120° C, drucklos oder unter Druck, kontinuierlich oder diskontinuierlich durchgeführt. Temperatur- und Druckbedingungen werden im allgemeinen so gewählt, daß die Umsetzung in der Flüssigphase stattfindet. Vorteilhaft führt man die Reaktion des ersten Schrittes während 0,5 bis 25, zweckmäßig 1 bis 18, vorzugsweise 1 bis 8 Stunden durch.

Die Umsetzung wird in Gegenwart katalytischer Mengen von Säure, vorteilhaft einer Menge von 0,1 bis 15, insbesondere von 0,5 bis 10, vorzugsweise 0,5 bis 5 Äquivalenten Säure, bezogen auf 1 Mol

Formamid, durchgeführt. Anstelle einbasischer Säuren können auch äquivalente Mengen mehrbasischer Säuren oder Stoffe, die unter den Reaktionsbedingungen solche Säuren bilden, zur Anwendung gelangen. Beispielsweise sind folgende Säuren oder ihre Ammoniumsalze geeignet: anorganische Säuren wie Chlorwasserstoff, Bromwasserstoff, Perchlorsäure, Schwefelsäure, Phosphorsäure, Salpetersäure; Sulfonsäuren wie Benzol- und p-Toluolsulfonsäure; Bor enthaltende Säuren wie Borsäure, Borfluorwasserstoffsäure; halogensubstituierte aliphatische Carbonsäuren wie Chloressigsäure, Dichloressigsäure, Trichloressigsäure, Monobromessigsäure, α- bzw. β-Chlorpropionsäure; oder entsprechende Gemische. Die Säuren können in konzentrierter Form, im Gemisch miteinander und/oder mit einem Lösungsmittel (insbesondere Wasser) angewendet werden. Bevorzugt sind Schwefelsäure, Phosphorsäure, p-Toluolsulfonsäure, Monochloressigsäure, Di-, Trichloressigsäure. Der pH der Umsetzung in Stufe 1 beträgt zweckmäßig von 1 bis 6,9, vorzugsweise von 2 bis 6.

Vorteilhaft kann man die Umsetzung ohne zusätzliche Lösungsmittel durchführen, wobei dann das Ausgangsgemisch bzw. Reaktionsgemisch als Medium (flüssige Phase) dient.

Ebenfalls kommen auch zusätzlich unter den Reaktionsbedingungen inerte, organische, nichtaromatische Lösungsmittel in Betracht. Als nichtaromatische Lösungsmittel kommen z. B. in Frage: Halogenkohlenwasserstoffe, insbesondere Chlorkohlenwasserstoffe, z. B. Tetrachloräthylen, 1,1,2,2- oder 1,1,1,2-Tetrachloräthan, Amylchlorid, Cyclohexylchlorid, Dichlorpropan, Methylenchlorid, Dichlorbutan, Isopropylbromid, n-Propylbromid, Butylbromid, Chloroform, Äthyljodid, Propyljodid, Tetrachlorkohlenstoff, 1,1,1- oder 1,1,2-Trichloräthan, Trichloräthylen, Pentachloräthan, 1,2-Dichloräthan, 1,1-Dichloräthan, n-Propylchlorid, 1,2-cis-Dichloräthylen, n-Butylchlorid, 2-, 3- und iso-Butylchlorid; Äther, z. B. Äthylpropyläther, Methyl-tert.-butyläther, n-Butyläthyläther, Di-n-butyläther, Diisobutyläther, Diisoamyläther, Diisopropyläther, Cyclohexylmethyläther, Diäthyläther, Äthylenglykoldimethyläther, Tetrahydrofuran, Dioxan; Ester wie Methylacetat, n-Propylacetat, Methylpropionat, Butylacetat, Äthylformiat, Essigester; Nitrokohlenwasserstoffe wie Nitromethan, Nitroäthan; Nitrile wie Fettsäurenitrile mit 2 bis 4 Kohlenstoffatomen, z. B. Acetonitril, Propionitril, Butyronitril, Isobutyronitril; Alkanole und Cycloalkanole wie Äthanol, Methanol, n-Butanol, Isobutanol, tert.-Butanol, Glykol, Glycerin, n-Propanol, Isopropanol, Amylalkohol, Cyclohexanol, 2-Methyl-4-pentanol, Äthylenglykolmonoäthyläther, 2-Äthylhexanol, Methylglykol, n-Hexanol, Isohexylalkohol, Isoheptylalkohol, n-Heptanol, Äthylbutanol, Nonylalkohol, Dodecylalkohol, Methylcyclohexanol, Diacetonalkohol, insbesondere solche mit 3 bis 8 Kohlenstoffatomen; Schwefelkohlenstoff; Sulfoxide wie Dimethylsulfoxid, Diäthylsulfoxid, Dimethylsulfon, Diäthylsulfon, Methyläthylsulfon, Tetramethylensulfon; aliphatische oder cycloaliphatische Kohlenwasserstoffe, z. B. Heptan, α-Pinen, Pinan, Nonan, Benzinfraktionen innerhalb eines Siedepunktintervalls von 50 bis 150°C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan; und entsprechende Gemische. Zweckmäßig verwendet man das Lösungsmittel in einer Menge von 10 bis 500 Gewichtsprozent, vorzugsweise von 20 bis 200 Gewichtsprozent, bezogen auf Ausgangsstoff II.

Die Reaktion des ersten Schrittes kann wie folgt durchgeführt werden: Ein Gemisch von Ausgangsstoff II, Formamid, Säure und gegebenenfalls Lösungsmittel wird während der Reaktionszeit bei der Reaktionstemperatur gehalten. Die Reaktionspartner können alle zu Beginn der Reaktion vollständig zusammengegeben oder teilweise während der Reaktion nachgegeben werden. Vorteilhaft wird das während der Reaktion gebildete Wasser aus dem Reaktionsgemisch entfernt, z. B. durch Abdestillieren im Vakuum, zweckmäßiger jedoch durch azeotrope Destillation. Als Schleppmittel dienen hierzu bei niedrigsiedenden Aldehyden diese selbst, bei höhersiedenden Aldehyden einige der vorgenannten, unter Reaktionsbedingungen inerten Lösungsmittel, wie Cyclohexan. Nach Beendigung der Reaktion wird der Endstoff in üblicher Weise, z. B. durch Destillation oder Kristallisation aus den Reaktionsgemischen abgetrennt.

Die Umsetzung wird im zweiten Schritt in der Gasphase bei einer Temperatur von 250 bis 700, vorzugsweise von 350 bis 500°C, drucklos, unter Überdruck oder zweckmäßig unter Unterdruck, vorzugsweise bei 0,1 bis 500 mbar, insbesondere 0,5 bis 200 mbar, kontinuierlich oder diskontinuierlich durchgeführt. Vorteilhaft können Trägergase, die unter Reaktionsbedingungen inert sind, z. B. Stickstoff, mitverwendet werden, zweckmäßig in einer Menge von 20 bis 80 Gewichtsprozent, bezogen auf die Gewichtsmenge Ausgangsstoff III. Bei Röhrenreaktoren wird vorzugsweise unter Unterdruck, bei Wirbelschichtreaktoren vorzugsweise bei Normaldruck mit Stickstoff als Trägergas gearbeitet.

Die Dehydratationskatalysatoren werden im allgemeinen in einer Menge von 0,01 bis 20, vorzugsweise 0,1 bis 10 Gewichtsprozent, bezogen auf Ausgangsstoff III, verwendet. Bei kontinuierlicher Verfahrensweise verwendet man in der Regel 1 bis 100, vorteilhaft 2 bis 50 Mol Ausgangsstoff III pro Stunde und Liter Katalysator. Als Katalysatoren werden im allgemeinen Säuren, z. B. Mineralsäuren wie Phosphorsäure und Schwefelsäure, nicht verwendet. Man verwendet zweckmäßig neutrale oder saure Dehydratationskatalysatoren. Vorteilhaft kommen als Katalysatoren in Frage: sauer aktivierte Bentonite wie Floridaerde und Fullererde, Bauxit, Ton, Kaolin, Bleicherde; Phosphorsäureester; Oxide von Aluminium, Silicium, Thorium, Zink, Wolfram, Titan, z. B. γ-Aluminiumoxid, Aktivtonerde, Aluminiumoxid aktiv, Kieselgel, blaues Wolframoxid, Titanweiß,

Anatas, Titandioxid/Aktivkohle, Aluminiumoxid/basisches Kupfercarbonat, Aluminiumoxid/Nickelcarbonat, Aluminiumoxid/Zinksulfid, Aluminiumoxid/Zinkoxid; Aluminiumhydroxide und -oxidhydrate wie Diaspor, Böhmit, Bayerit A und B, Hydrargillit; OXidgemische vorgenannter Oxide, z. B. Aluminiumoxid mit Kupferoxid, Zirkonoxid, Wolframoxid, Chromoxid, Thoriumoxid, Cer-(IV)-oxid, Molybdän-(IV)-oxid, Nickeloxid, Cobaltoxid, Siliciumoxid/Magnesiumoxid/Tantal-V-oxid, Siliciumoxid/Magnesiumoxid/Chrom-III-oxid, Silikat- bzw. Kieselsäuregel/Metalloxid der III. bis VIII. Gruppe des Periodischen Systems wie Aluminium, Titan, Vanadium, Chrom, Mangan, Tantal; Chloride, Bromide, Sulfate, Hydrogensulfate, Pyrosulfate, Phosphate, Pyrophosphate, Borate von Lithium, Natrium, Kalium, Kupfer, Magnesium, Zink, Bor, Calcium, Aluminium, z. B. Zinksulfat, Borphosphat, Natriumalaun, Kaliumalaun, Dinatriumphosphat, Tricalciumphosphat, Magnesiumpyrophosphat, Nickelphosphat, Aluminiumphosphat, Natriumphosphat/Graphit, Aluminiumphosphat/Bims bzw. Gemische wie Kupferphosphat/Lithiumphosphat/Eisenphosphat, Natriumphosphat/Butylaminphosphat, Kupfer-I-bromid/Ammoniumbromid. Bezüglich der Herstellung von Dehydratationskatalysatoren wird auf Houben-Weyl, Methoden der Organischen Chemie, Band 4/2, Seiten 209 bis 218, und die deutsche Offenlegungsschrift 2 036 503 verwiesen.

Vorteilhaft verwendet man im zweiten Schritt keine zusätzlichen organischen Lösungsmittel, gegebenenfalls kommen aber die schon für den ersten Schritt vorgenannten, inerten, nichtaromatischen Lösungsmittel in Betracht, vorzugsweise die vorgenannten Carbonsäurenitrile oder auch aromatische Kohlenwasserstoffe und Carbonsäurenitrile wie Toluol, Benzol, Hylole; Benzonitril, o-Tolunitril, p-Tolunitril, Äthylbenzonitril. Zweckmäßig verwendet man das Lösungsmittel in einer Menge von 10 bis 1000 Gewichtsprozent, vorzugsweise von 20 bis 500 Gewichtsprozent, bezogen auf Ausgangsstoff III.

Die Reaktion kann wie folgt durchgeführt werden: Der Ausgangsstoff III wird nach Verdampfen über den auf die Reaktionstemperatur erhitzten Katalysator in einem Röhrenreaktor geleitet. Eine Verweilzeit von 0,001 bis 40, insbesondere 0,01 bis 20 Sekunden im Reaktionsraum ist vorteilhaft. Aus dem den Reaktor verlassenden Reaktionsgemisch wird der Endstoff in üblicher Weise, z. B. durch fraktionierte Destillation, isoliert. Man kann aber auch dem Reaktionsgemisch Proben entnehmen, durch analytische, z. B. gaschromatographische Bestimmung des Verhältnisses von Endstoff I und Ausgangsstoff III im Reaktionsgemisch den Umsatz feststellen und das Reaktionsgemisch ohne Abtrennung des Endstoffes direkt weiterverarbeiten, z. B. zu den entsprechenden gesättigten Nitrilen oder Aminen hydrieren.

In einer bevorzugten Ausführungsform des Verfahrens werden die Ausgangsstoffe in einer Wirbelschicht bei der Reaktionstemperatur umgesetzt. Der Katalysator bzw. Katalysator auf Träger kann zweckmäßig durch Inertgas, einem Gemisch von Ausgangsstoff III und Inertgas oder dem Ausgangsstoff III allein als Wirbelschichtgas bei Normaldruck oder erhöhtem oder insbesondere vermindertem Druck in einer Wirbelschicht gehalten werden. Entsprechend kann die Gesamtmenge oder eine Teilmenge an Ausgangsstoff III getrennt von dem Wirbelschichtgas in den Wirbelschichtreaktor eingeleitet werden. Der Ausgangsstoff III kann auch in einem beheizten Vorratsgefäß flüssig gehalten und in einen Verdampfer, der dem Wirbelschichtreaktor vorgeschaltet ist, dosiert werden. Gleichzeitig leitet man vorteilhaft einen schwachen Stickstoffstrom, zweckmäßig von 100 bis 1000 Volumenteilen Stickstoff je Stunde und Volumenteil Katalysator, durch den Verdampfer. Die verdampften Ausgangsstoffe werden zusammen mit dem Stickstoffstrom durch das Katalysatorbett geleitet. Die Konzentration des Ausgangsstoffes III im Inertgas beträgt vorteilhaft 0,1 bis 50 Volumenprozent. Man kann das Verfahren nach der Erfindung in einem einfachen oder unterteilten, offenen oder geschlossenen Wirbelschichtsystem mit und ohne Fließstaubzirkulation durchführen. Bezüglich Reaktoren, Durchführung, Verfahrensvarianten und Reaktionsbedingungen des Wirbelschichtverfahrens wird auf Ullmanns Encyklopädie der technischen Chemie, Band 1, Seiten 916 ff, verwiesen. Die Aufarbeitung des Reaktionsgemisches erfolgt in vorgenannter Weise.

Die nach dem Verfahren der Erfindung herstellbaren Nitrile I sind wertvolle Ausgangsstoffe für die Herstellung von Farbstoffen, Pflanzenschutzmitteln, Weichmachern und Pharmazeutika. Durch Hydrierung an Palladiumkatalysatoren, zweckmäßig auf Trägern wie z. B. Kohle oder $SiO_2$, liefern sie gesättigte Nitrile. Bezüglich der Verwendung wird auf die vorgenannten Veröffentlichungen und Ullmanns Encyklopädie der technischen Chemie, Band 12, Seiten 751 bis 764, verwiesen.

Die in den Beispielen genannten Teile sind Gewichtsteile. Die Gewichtsteile verhalten sich zu den Volumenteilen wie Kilogramm zu Liter.

## Beispiel 1

a) (1. Schritt) In einer mit Rückflußkühler und Wasserabscheider versehenen Rührapparatur werden 1440 Teile Isobutyraldehyd, 900 Teile Formamid und 240 Teile Toluolsulfonsäure vorgelegt und 15 Stunden unter Rückfluß erhitzt, wobei durch portionsweise Zugabe von weiteren 245 Teilen Isobutyraldehyd die Innentemperatur während der Reaktion im Bereich von 110°C gehalten wird. Es scheiden sich 318 Teile Wasser ab. Bei der anschließenden Destillation des Reaktionsgemisches vim Vakuum werden 1527 Teile N-(2-Methyl-1-propenyl)-formamid vom Kp 0,4 mbar 78 bis

81° C entsprechend einer Ausbeute von 77,1% der Theorie, bezogen auf eingesetztes Formamid, erhalten.

b) (2. Schritt) 105 Teile N-(2-Methyl-1-propenyl)-formamid werden pro Stunde aus einem Vorratsgefäß in einen auf 220° C erhitzten horizontalen Quarzverdampfer dosiert und der Dampf zusammen mit 37 600 Volumenteilen pro Stunde Stickstoff durch den auf 500° C erhitzten Wirbelreaktor geleitet. Der Wirbelreaktor ist ein vertikal auf dem Verdampfer sitzendes, elektrisch beheiztes Quarzrohr, das nach unten mit einer eingeschmolzenen Quarzfritte abgeschlossen ist. Das Quarzrohr ist mit 100 Volumenteilen eines Katalysators aus Kieselgel (Schüttgewicht 420 g/l, spez. Wasseraufnahme 1,39 $cm^3/g$, Korngröße 0,1 bis 0,3 mm) zu einem Drittel gefüllt. Die Verweilzeit in der Katalysatorzone im Wirbelzustand beträgt 3,5 Sekunden. Die Höhe der Katalysatorzone beträgt im Wirbelzustand 80 mm. Die den Reaktor verlassenden Dämpfe werden kondensiert und fraktioniert destilliert. Man erhält stündlich 58 Teile (67,5% der Theorie, bezogen auf eingesetztes N-(2-Methyl-1-propenyl)-formamid) 3,3-Dimethylacrylnitril vom Kp 80° C (153 mbar). Der Umsatz ist praktisch quantitativ, bezogen auf eingesetztes N-(2-Methyl-1-propenyl)-formamid. Die Ausbeute blieb auch noch nach 300 Stunden Betrieb konstant.

## Beispiel 2

Man verfährt wie in Beispiel 1, setzt jedoch in 1b) als Katalysator Kieselgel der Korngröße 0,1 bis 0,3 mm und eines Schüttgewichtes 540 g/l (spez. Wasseraufnahme 0,92 $cm^3/g$) in einem Rohrreaktor (Verhältnis Länge zu Durchmesser 3,4 zu 1) ein. In den auf 450 bis 480° C vorgeheizten und auf 133 mbar evakuierten Reaktor werden stündlich 158,5 Teile N-(2-Methyl-1-propenyl)-formamid eingegeben. Man erhält nach Destillation 95,9 Teile 3,3-Dimethylacrylnitril pro Stunde (74% der Theorie, bezogen auf eingesetztes N-(2-Methyl-1-propenyl)-formamid) vom Kp 80° C (153 mbar). Der Umsatz, bezogen auf Ausgangsstoff III, ist praktisch quantitativ.

## Beispiel 3

a) (1. Schritt) In einer mit Rückflußkühler und Wasserabscheider versehenen Rührapparatur werden 1075 Teile 2-Methylbutanal, 450 Teile Formamid und 47,5 Teile p-Toluolsulfonsäure vorgelegt und 6 Stunden unter Rückfluß erhitzt, wobei durch portionsweise Zugabe von weiteren 235 Teilen 2-Methylbutanal die Innentemperatur während der Reaktion im Bereich von 92 bis 100° C gehalten wird. Es scheiden sich 107 Teile Wasser ab. Bei der anschließenden Destillation des Reaktionsgemisches werden neben 704 Teilen unumgesetztem 2-Methylbutanal 641 Teile N-(2-Methyl-1-butenyl)-formamid vom Kp (0,35 mbar) 84° C entsprechend einer Ausbeute von 80,5%, bezogen auf umgesetztes 2-Methylbutanal, erhalten.

b) (2. Schritt) Das in Beispiel 2 beschriebene Reaktionsrohr (Länge : Durchmesser = 1,7 : 1; 50 Volumenteile Katalysator) enthält nur eine 5,5 cm hohe Katalysatorzone. Man setzt analog Beispiel 2 bei 430° C und 133 mbar stündlich 107,1 Teile N-(2-Methyl-1-butenyl)-formamid um. Der Ausgangsstoff ist praktisch vollständig umgesetzt. Man erhält 68,6 Teile pro Stunde 3-Methyl-pent-2-ennitril vom Kp (152 mbar) 87 bis 94° C, entsprechend einer Ausbeute von 76,1% der Theorie, bezogen auf eingesetztes N-(2-Methyl-1-butenyl)-formamid.

## Beispiel 4

a) (1. Schritt) In einer mit Rückflußkühler und Wasserabscheider versehenen Rührapparatur werden 500 Teile 2-Methylpentanal, 180 Teile Formamid und 38 Teile p-Toluolsulfonsäure zusammen mit 200 Volumenteilen Cyclohexan 3½ Stunden bei 98° C unter Rückfluß erhitzt, wobei sich 68 Teile Wasser abscheiden. Bei der anschließenden Destillation des Reaktionsgemisches werden neben dem Cyclohexan und 131 Teilen unumgesetztem 2-Methylpentanal 319 Teile N-(2-Methyl-1-pentenyl)-formamid vom Kp (0,4 mbar) 100 bis 101° C, entsprechend einer Ausbeute von 68,1 Prozent, bezogen auf umgesetztes 2-Methylpentanal, erhalten.

b) (2. Schritt) Analog Beispiel 3 werden stündlich bei 490° C und 133 mbar 105,3 Teile N-(2-Methyl-1-pentenyl)-formamid umgesetzt. Man erhält 72,6 Teile 3-Methylhex-2-ennitril pro Stunde vom Kp (153 mbar) 112 bis 114° C, entsprechend einer Ausbeute von 80,4 Prozent, bezogen auf eingesetztes N-(2-Methyl-1-pentenyl)-formamid.

Beispiel 5

a) (1. Schritt) In einer mit Rückflußkühler und Wasserabscheider versehenen Rührapparatur werden 768 Teile 2-Äthylhexanal, 225 Teile Formamid und 24 Teile p-Toluolsulfonsäure zusammen mit 600 Volumenteilen Cyclohexan 6 1/2 Stunden bei 92 bis 95°C unter Rückfluß erhitzt, wobei sich 81 Teile Wasser abscheiden. Das Reaktionsgemisch wird fraktioniert destilliert. Neben 393 Teilen unumgesetztem 2-Äthylhexanal erhält man 339 Teile N-(2-Äthyl-1-hexenyl)-formamid vom Kp (0,7 mbar) 117°C, entsprechend einer Ausbeute von 74,7% der Theorie, bezogen auf umgesetztes 2-Äthylhexanal.

b) (2. Schritt) Analog Beispiel 3 werden bei 410°C und 67 mbar stündlich 51,9 Teile N-(2-Äthyl-1-hexenyl)-formamid umgesetzt. Man erhält 28,2 Teile 3-Äthyl-hept-2-ennitril vom Kp (33 mbar) 96°C, entsprechend einer Ausbeute von 77,3% der Theorie, bezogen auf eingesetztes N-(2-Äthyl-1-hexenyl)-formamid.

Beispiel 6

a) (1. Schritt) In einer mit Rückflußkühler und Wasserabscheider versehenen Rührapparatur werden 1340 Teile 2-Phenylpropanal, 450 Teile Formamid und 95 Teile p-Toluolsulfonsäure zusammen mit 420 Volumenteilen Cyclohexan 4 1/2 Stunden bei 95 bis 98°C unter Rückfluß erhitzt, wobei sich 144 Teile Wasser abscheiden. Das Reaktionsgemisch wird fraktioniert destilliert. Das Destillat wird mit 500 Volumenteilen Äther extrahiert. Aus dem Ätherextrakt erhält man durch Vakuumdestillation 118 Teile unumgesetztes 2-Phenylpropanal und aus dem Extraktionsrückstand und Destillationsrückstand durch Kristallisation 947 Teile N-(2-Phenyl-1-propenyl)-formamid, entsprechend einer Ausbeute von 64,5% der Theorie, bezogen auf umgesetztes 2-Phenylpropanal.

b) (2. Schritt) Analog Beispiel 3 werden bei 500°C und 0,4 mbar stündlich 69 Teile bei 130°C geschmolzenes N-(2-Phenyl-1-propenyl)-formamid umgesetzt. Man erhält pro Stunde 44 Teile 3-Phenyl-but-2-ennitril vom Kp (0,5 mbar) 90 bis 91°C, entsprechend einer Ausbeute von 71,9% der Theorie, bezogen auf eingesetztes N-(2-Phenyl-1-propenyl)-formamid.

**Patentanspruch**

Verfahren zur Herstellung von $\alpha,\beta$-ungesättigten Nitrilen der Formel

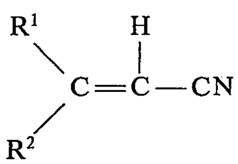

(I)

worin $R^1$ und $R^2$ gleich oder verschieden sein können und jeweils einen aliphatischen, cycloaliphatischen, araliphatischen, aromatischen oder heterocyclischen Rest bedeuten, $R^1$ und $R^2$ zusammen mit dem benachbarten Kohlenstoffatom auch Glieder eines Ringes bezeichnen, durch Umsetzung von Aldehyden mit Säureamiden in Gegenwart von sauren Katalysatoren und Lösungsmitteln, dadurch gekennzeichnet, daß man in einem ersten Schritt einen Aldehyd der Formel

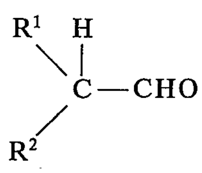

(II)

worin $R^1$ und $R^2$ die vorgenannten Bedeutungen besitzen, mit Formamid in Gegenwart von katalytischen Mengen einer anorganischen Säure, Sulfonsäure und/oder halogensubstituierten, aliphatischen Carbonsäure in Abwesenheit von zusätzlichen Lösungsmitteln oder in Gegenwart unter den Reaktionsbedingungen inerter, organischer, nichtaromatischer Lösungsmittel umsetzt und dann in einem zweiten Schritt das so gebildete N-Alkenylformamid der Formel

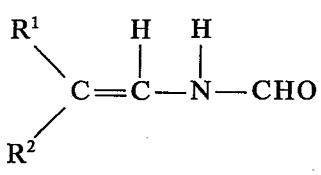

(III)

worin $R^1$ und $R^2$ die vorgenannten Bedeutungen besitzen, in der Gasphase bei einer Temperatur von 250 bis 700° C in Gegenwart eines Dehydratationskatalysators umsetzt.

**Claim**

A process for the preparation of $\alpha,\beta$-unsaturated nitriles of the formula

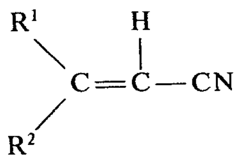

(I)

where $R^1$ und $R^2$ may be identical or different and each is an aliphatic, cycloaliphatic, araliphatic, aromatic or heterocyclic radical, and $R^1$ and $R^2$ together with the adjacent carbon may also be members of a ring, by reacting aldehydes with acid amides in the presence of acidic catalysts and solvents, characterized in that, in a first step, an aldehyde of the formula

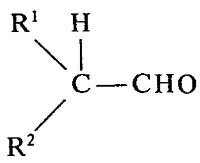

(II)

where $R^1$ and $R^2$ have the above meanings, is reacted with formamide in the presence of a catalytic amount of an inorganic acid, sulfonic acid and/or halogen-substituted aliphatic carboxylic acid in the absence of an added solvent or in the presence of an organic non-aromatic solvent which is inert under the reaction conditions, after which, in a second step, the resulting N-alkenylformamide of the formula

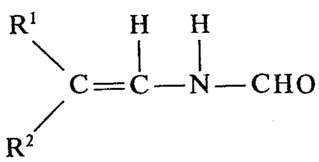

(III)

where $R^1$ and $R^2$ have the above meanings, is passed, in the gas phase, over a dehydration catalyst at from 250 – 700° C.

**Revendication**

Procédé pour la préparation de nitriles $\alpha,\beta$-insaturés de formule

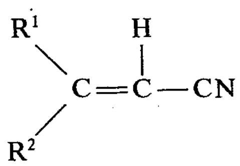

(I)

dans laquelle $R^1$ et $R^2$ peuvent être semblables ou différents et représentent chacun un radical aliphatique, cycloaliphatique, araliphatique, aromatique ou hétérocyclique, $R^1$ et $R^2$ formant également, avec l'atome de carbone voisin, des termes d'un noyau, par réaction d'aldéhydes avec des amides d'acides en présence de catalyseurs acides et de solvants, caractérisé en ce qu'en un premier temps, on fait réagir un aldéhyde de formule

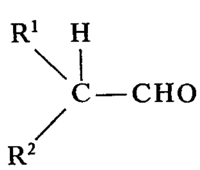

(II)

dans laquelle $R^1$ et $R^2$ ont les significations données ci-dessus, avec un formamide en présence de quantités catalytiques d'un acide minéral, d'un acide sulfonique et/ou d'un acide carboxylique aliphatique halogénosubstitué, en l'absence de solvants supplémentaires ou en présence de solvants

organiques non aromatiques, inertes dans les conditions de la réaction, puis, en un second temps, on fait réagir le N-alcénylformamide ainsi formé, de formule

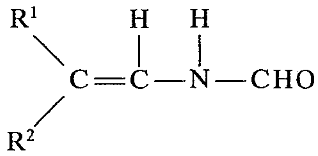

(III)

dans laquelle $R^1$ et $R^2$ ont les significations données ci-dessus, en phase gazeuse, à une température de 250 à 700° C, en présence d'un catalysateur de déshydratation.